# EUROPÄISCHE PATENTSCHRIFT

(11) **EP 2 501 293 B1**
(45) Veröffentlichungstag und Bekanntmachung des Hinweises auf die Patenterteilung: **22.10.2014**
(21) Anmeldenummer: 10787049.5
(22) Anmeldetag: 15.11.2010
(51) Int. Cl.: A61B 6/10, E06B 3/48, E06B 5/18, G21F 3/00

(54) **STRAHLENSCHUTZ-LAMELLENANORDNUNG**
SLAT ARRANGEMENT FOR PROTECTION AGAINST RADIATION
AGENCEMENT DE LAMELLES POUR RADIOPROTECTION

(30) Priorität: 17.11.2009 DE 102009053619
(43) Veröffentlichungstag der Anmeldung: 26.09.2012
(73) Patentinhaber: Mavig GmbH, 81829 München (DE)
(72) Erfinder: SCHULZ, Jürgen, 85521 Riemerling (DE); BALLSIEPER, Barbara, 81829 München (DE)
(74) Vertreter: Vossius & Partner
(86) Internationale Anmeldenummer: PCT/EP2010/067437
(87) Internationale Veröffentlichungsnummer: WO 2011/061135

(56) Entgegenhaltungen:
- EP-A2- 1 126 125
- DE-A1- 1 466 848
- DE-A1- 10 240 151
- DE-U1- 20 112 678
- IT-B- 1 069 157
- US-A- 4 062 518
- US-A- 5 900 638
- US-A1- 2008 025 470
- US-A1- 2009 020 713

## Beschreibung

Die vorliegende Erfindung betrifft eine Strahlenschutz-Lamellenanordnung, insbesondere zur Abschirmung der durch eine Röntgenstrahlungsquelle abgestrahlten Röntgenstrahlung, welche beispielsweise bei Röntgenuntersuchungen zum Einsatz kommt.

Um die durch Röntgenuntersuchung hervorgerufenen Strahlungsbelastungen für die beteiligten Personen möglichst niedrig zu halten, ist es unter anderem aufgrund der gängigen Vorschriften Praxis, Röntgenräume derart auszugestalten, dass keine oder nur minimale Strahlung aus dem Röntgenraum austritt. Hierzu werden üblicherweise röntgenundurchlässige Materialien bei der Errichtung des Röntgenraums verwendet.

Ferner sind verschiedene Arten von Strahlenschutzvorrichtungen gegen Röntgenstrahlen bekannt. So zeigt beispielsweise die DE 1 959 358 A einen Strahlenschutz gegen Röntgenstrahlen in Form eines Strahlenschutzvorhangs an einer verstellbaren Halterung. Der Strahlenschutzvorhang besteht aus mehreren, nebeneinander angeordneten, sich gegenseitig überlappenden Streifen, die zum Beispiel Blei enthalten können. Die Halterung weist eine Tragstange für den Strahlenschutzvorhang auf, die längs verschiebbar und vorzugsweise in jeder Position festlegbar angebracht ist. Die Tragstange kann in sich biegsam ausgebildet sein und gegebenenfalls schwenkbar mit einer Schiene verbunden sein.

Ferner offenbart die DE 27 49 826 A1 ein Röntgenstrahl-Abschirmsystem mit einzelnen Lamellen, die um ihre Längsachse jeweils verschwenkbar an einer Trägereinrichtung angeordnet sind. Dabei sind die Lamellen jeweils einzeln unabhängig voneinander aufgehängt.

Eine ähnliche Anordnung ist in der DE 83 03 847 U1 beschrieben. Auch dort sind Lamellen für Vorhänge zur Abschirmung von Röntgenstrahlung offenbart, wobei die Lamellen aus einer durchgehenden Bleiplatte mit beidseits beschichtetem Kunststoffmaterial bestehen.

Auch die DE 93 13 473 U1 beschreibt einen horizontal verstellbaren Lamellenschutzvorhang gegen Röntgenstrahlen. Der Vorhang kann in eine geöffnete und eine geschlossene Stellung gebracht werden. Die Lamellen sind einzeln aufgehängt. Dazu werden die Lamellen mit einer rechteckigen Aufnahmeöffnung am oberen Ende in Lamellenhaken eingehängt. In einer Führungs- und Befestigungsschiene ist ein Bewegungsmechanismus vorgesehen, mit dem die Lamellen entlang der Schiene verschoben werden können und entlang der die Lamellen um 180° drehbar sind. Der Bewegungsmechanismus ist über Schnüre oder einen Elektromotor betätigbar. Die einzelnen Lamellen bestehen aus einer plattenförmigen Bleieinlage, die beidseitig mit Kunststoff abgedeckt ist.

Die DE 200 08 768 U1 beschreibt eine Schirmwand, insbesondere gegen radioaktive Strahlung, die aus formschlüssig ineinandergreifenden stapelbaren Elementen besteht. Eine Verschiebung der einzelnen Elemente ist nicht vorgesehen. Vielmehr handelt es sich um eine stationär stehende Schirmwand.

Die DE 200 13 658 U1 beschreibt einen Strahlenschutz-Lamellenvorhang, bei dem einzelne Lamellen jeweils einem Laufwagen zugeordnet sind. An diesem Laufwagen ist die Lamelle um ihre Längsachse drehbar aufgehängt. Alle Laufwagen sind in einem Tragprofil aufgereiht angeordnet, wobei jeweils zwei benachbarte Laufwagen jeweils als Scherengelenk mit zwei an einem ihrer Enden gelenkig miteinander verbundenen und an ihrem jeweils anderen Ende gelenkig an einem der Laufwagen befestigten Gelenkhebeln ausgebildet sind. Die Gelenkhebel jedes Scherengelenks sollen zwischen einer ersten Endstellung mit spitzem Winkel und einer zweiten Endstellung mit stumpfem Winkel verstellbar sein. Der Strahlenschutz-Lamellenvorhang weist zwei Antriebssysteme auf, wobei ein erstes zum Öffnen und Schließen des Vorhangs und ein zweites zum Verstellen der Lamellen vorgesehen ist. Eine ähnliche Konstruktion ist in der DE 201 12 678 U1 offenbart.

Eine weitere Strahlenschutzvorrichtung ist beispielsweise in der DE 297 06 321 U1 beschrieben. Diese besteht aus einem oberen und einem unteren Teil. Der obere Teil weist eine optisch transparente Schutzscheibe auf, während der untere Teil aus einem flexiblen Material besteht, der einen Schutzvorhang bildet. Die Schutzscheibe kann gegenüber der Vertikalen in Richtung zum Patienten geneigt sein. Die den Schutzvorhang bildenden Lamellen können ein Band aufweisen, mit dem jede Lamelle an einem an der Befestigungsschiene vorgesehenen Gegenstück befestigt werden kann.

Die DE 297 22 765 U1 betrifft eine Lamelle für einen Strahlenschutz-Lamellenvorhang. Es wird unter anderem vorgeschlagen, eine Bleieinlage in einen Hohlraum einzulegen und darin festzuhalten.

Die WO 2006/079472 A1 beschreibt ein Strahlenschutztor, insbesondere für eine Röntgenprüfanlage, wie sie beispielsweise an Flughäfen zum Einsatz kommt. Bei diesem Strahlenschutztor sind Lamellen vorgesehen, deren Stirnseiten Hebel darstellen, die nach Art einer "Nürnberger Schere" verbunden sein sollen. Die Lamellen sind zwischen einer geöffneten Stellung und einer geschlossenen Stellung verstellbar. An einer Kante einer Lamelle kann ein Bleilappen vorgesehen sein, der gewährleisten soll, dass im geschlossenen Zustand des Tores der Übergangsbereich zwischen zwei Lamellen überdeckt ist.

Es ist eine Aufgabe der vorliegenden Erfindung, eine verbesserte Strahlenschutz-Lamellenanordnung bereitzustellen, die einen verbesserten Strahlenschutz und flexiblen Einsatz ermöglicht. Diese Aufgabe wird mit den Merkmalen der Patentansprüche gelöst.

Die Erfindung geht dabei von dem Grundgedanken aus, eine Strahlenschutz-Lamellenanordnung nach dem Prinzip eines Faltvorhangs bereitzustellen, so dass eine im Wesentlichen vollständig geschlossene Strahlenschutzfläche bereitgestellt wird. Hierzu weist die Strahlenschutz-Lamellenanordnung eine Führung und mehrere entlang der Führung beweglich angeordnete Strahlenschutz-Lamellen auf. Die Anordnung kann von einem offenen Zustand durch Verschieben und Rotieren der Lamellen in einen geschlossenen Zustand überführt werden, wobei aneinander angrenzende Lamellen einander überlappen und gelenkig miteinander verbunden sind, um den Faltvorhang zu bilden.

Durch die überlappende und gelenkig verbundene Anordnung benachbarter Lamellen ist sichergestellt, dass die erfindungsgemäße Strahlenschutz-Lamellenanordnung einen Strahlenaustritt verhindert. Ferner ist durch die Verbindung der einzelnen Lamellen miteinander gewährleistet, dass keine Gegenstände zwischen die Lamellen geraten können und so ein unbeabsichtigtes Austreten von Strahlung effektiv verhindert werden kann.

Vorzugsweise sind die Lamellen derart überlappend angeordnet, dass in jeder Position und/oder Orientierung der Lamellen eine im Wesentlichen geschlossene Strahlenschutzfläche gebildet wird. Dies hat insbesondere den Vorteil, dass ein und dieselbe Strahlenschutz-Lamellenanordnung für verschieden große Verfahrwege eingesetzt werden kann. Dadurch ist ein flexibler Einsatz sichergestellt. Bevorzugt schließen die aneinander angrenzenden Lamellen einen Winkel ein, der zwischen 5° (geöffneter Strahlenschutz) und 160° (geschlossener Strahlenschutz), bevorzugt zwischen 15° und 145°, variieren kann. Dabei sind die Lamellen bevorzugt derart überlappend angeordnet, dass bei Winkeln größer als 100°, bevorzugt bei Winkeln größer als 120°, eine geschlossene Strahlenschutzfläche gebildet wird. Bevorzugt wird ferner eine geschlossene Strahlenschutzfläche gebildet, wenn die eingeschlossenen Winkel kleiner als 80°, bevorzugt kleiner als 60°, sind.

Es ist ferner bevorzugt, mindestens eine Längsseite einer jeweiligen Lamelle abgewinkelt auszubilden. Dabei korrespondieren vorzugsweise der Winkel innerhalb der Lamelle und der zwischen angrenzenden Lamellen gebildete Maximalwinkel so, dass bei vollständig geschlossenem Strahlenschutz die angrenzende Lamelle im Wesentlichen parallel zu dem abgewinkelten Bereich zu liegen kommt. Vorzugsweise sind zwei aneinandergrenzende Lamellen in Form und/oder Größe unterschiedlich ausgebildet. Dabei ist es bevorzugt, eine erste, breitere Lamelle zu verwenden, die an ihren beiden gegenüberliegenden Längsseiten in entgegengesetzte Richtungen abgewinkelt ist. Benachbart zu einer derartigen breiten Lamelle ist eine zweite, schmälere Lamelle angeordnet, die vorzugsweise nicht abgewinkelt ist.

Aneinander angrenzende Lamellen sind vorzugsweise durch lösbar angebrachte Gelenkbolzen miteinander verbunden. Ein derartiger modularer Aufbau erlaubt das einzelne Auswechseln eventuell beschädigter Lamellen, wodurch kurze Stillstandszeiten gewährleistet werden können.

Jede der Lamellen weist vorzugsweise zwei Deckelemente auf, zwischen denen ein Hohlraum zur Aufnahme eines strahlungsundurchlässigen Materials und eines optionalen Füllmaterials gebildet ist. Alternativ ist es möglich, die Lamellen als geschlossene Hohlform auszubilden, in die das strahlungsundurchlässige Material eingebracht wird. Die beiden Deckelemente der Lamellen sind vorzugsweise im Wesentlichen identisch ausgebildet und punktsymmetrisch zueinander angeordnet. Dadurch können mit einem einzigen Formwerkzeug die beiden Deckelemente baugleich hergestellt werden. Es ist bevorzugt, dass die beiden Deckelemente, das strahlenundurchlässige Material und das optionale Füllmaterial miteinander verklebt sind. Zusätzlich oder alternativ können die Deckelemente formschlüssig oder durch zusätzliche Verbindungsmittel miteinander verbunden sein.

Die Führung für die Strahlenschutz-Lamellen ist vorzugsweise als Aufhängung für die Lamellen ausgebildet. Bevorzugt sind die Lamellen jeweils über ein Kugel- oder Kreuzgelenk an der Führung angebracht. Dies hat insbesondere den Vorteil, dass im Falle eines Auslenkens der Lamellen, beispielsweise wenn jemand gegen eine oder mehrere der Lamellen stößt, die Führung nicht beschädigt wird, sondern die Lamellen ausgelenkt werden können. Die Führung zum Verschieben und Rotieren der Lamellen weist vorzugsweise eine Nürnberger Schere auf.

Die Strahlenschutz-Lamellen werden vorzugsweise mit Hilfe eines Motors angetrieben. Dabei führen die Lamellen aufgrund ihrer Führung und Verbindung mit dem Motor zeitgleich eine translatorische und rotatorische Bewegung aus. Alternativ oder zusätzlich kann der Antrieb manuell erfolgen. Hierzu kann beispielsweise ein Kurbelmechanismus vorgesehen sein. Vorzugsweise ist der manuelle Betriebsmodus als Notfallbetätigung vorgesehen, so dass die Strahlenschutz-Lamellen auch bei einem Defekt, beispielsweise einem Stromausfall, geöffnet werden können.

Bevorzugt weist die Anordnung ferner eine Endlagenverriegelung auf, die es gestattet, die Lamellen im offenen und/oder geschlossenen Zustand zu fixieren bzw. verriegeln. Die Endlagenverriegelung kann beispielsweise einen oder mehrere Magnete aufweisen, mit Hilfe derer die Endlamelle z.B. an eine angrenzende Wand fixiert wird. Alternativ können aber auch mechanische Riegel bzw. Verschlüsse Verwendung finden, z.B. ein Rastmechanismus wie er bei Kugelschreibern angewandt wird.

Vorteilhafterweise kann die erfindungsgemäße Strahlenschutz-Lamellenanordnung mit einer Einrichtung zum Kommunizieren mit einem Röntgengerät ausgestattet sein, so dass das Röntgengerät nur dann betriebsbereit ist, wenn die erfindungsgemäße Strahlenschutz-Lamellenanordnung im geschlossenen Zustand ist und ein entsprechendes Signal an das Röntgengerät übermittelt wird. Ein derartiges Signal kann erfindungsgemäß auf einfache Weise erzeugt werden, nämlich immer dann wenn die Strahlenschutz-Lamellenanordnung in ihrer geschlossenen Stellung ist. Hierzu kann beispielsweise ein Endschalter verwendet werden, der nicht nur den Antrieb abschaltet, sondern gleichzeitig auch dem Röntgengerät mitteilt, dass die Anordnung geschlossen ist. Da die einzelnen Lamellen erfindungsgemäß so überlappend und gelenkig miteinander verbunden angeordnet sind, dass sie eine geschlossene Strahlenschutzfläche bilden, kann ein derart einfacher Kontrollmechanismus trotzdem sicher gewährleisten, dass keine Strahlung durch die Strahlenschutzanordnung austreten kann.

Obwohl die Erfindung insbesondere im Zusammenhang mit Röntgenstrahlung beschrieben wird, ist sie auch zur Abschirmung anderer Strahlungen geeignet, z.B. Laserstrahlung.

Die Erfindung wird nachfolgend unter Bezugnahme auf die Figuren anhand einer bevorzugten Ausführungsform näher beschrieben. Es zeigen:
- Figur 1A: eine Vorderansicht, Seitenansicht und Draufsicht einer erfindungsgemäßen Strahlenschutz-Lamellenanordnung im geschlossenen Zustand;
- Figur 1B: eine Vorderansicht, Seitenansicht und Draufsicht der Strahlenschutz-Lamellenanordnung von Figur 1A im geöffneten Zustand;
- Figur 2A: eine ausschnittsweise perspektivische Detailansicht einer erfindungsgemäßen Strahlenschutz-Lamellenanordnung im geschlossenen Zustand;
- Figur 2B: eine perspektivische Detailansicht ähnlich Figur 2A, wobei die Strahlenschutz-Lamellenanordnung geöffnet dargestellt ist;
- Figur 3A: eine perspektivische Detailansicht einer Antriebsseite der erfindungsgemäßen Strahlenschutz-Lamellenanordnung ähnlich Figur 2A, jedoch mit abgenommenem Gehäusedeckel;
- Figur 3B: eine Ansicht ähnlich Figur 3A, die ein der Antriebsseite gegenüberliegendes Ende näher zeigt;
- Figur 3C: eine Ansicht ähnlich Figur 3A, jedoch im geöffneten Zustand;
- Figur 4A: eine ausschnittsweise perspektivische Ansicht des oberen Endes der Lamellen mit Führungs- und Antriebselementen im geschlossenen Zustand;
- Figur 4B: eine Ansicht ähnlich Figur 4A im geöffneten Zustand;
- Figur 5: eine perspektivische Ansicht einer in der erfindungsgemäßen Strahlenschutz-Lamellenanordnung verwendeten Antriebs- und Getriebeeinrichtung;
- Figur 6A: eine auseinandergezogene Seitenansicht der Einzelkomponenten eines ersten Lamellentyps;
- Figur 6B: eine auseinandergezogene Seitenansicht eines zweiten Lamellentyps;
- Figur 6C: eine Querschnittsansicht durch die Lamelle gemäß Figur 6A;
- Figur 7: eine Seitenansicht eines Gehäuses zur Aufnahme der Antriebs- und Führungsteile der erfindungsgemäßen Strahlenschutz-Lamellenanordnung;
- Figur 8: eine Draufsicht einer erfindungsgemäßen Strahlenschutz-Lamellenanordnung im geschlossenen Zustand;
- Figur 9: eine Draufsicht einer erfindungsgemäßen Strahlenschutz-Lamellenanordnung im teilweise geschlossenen Zustand; und
- Figur 10: eine Draufsicht einer erfindungsgemäßen Strahlenschutz-Lamellenanordnung im geöffneten Zustand.

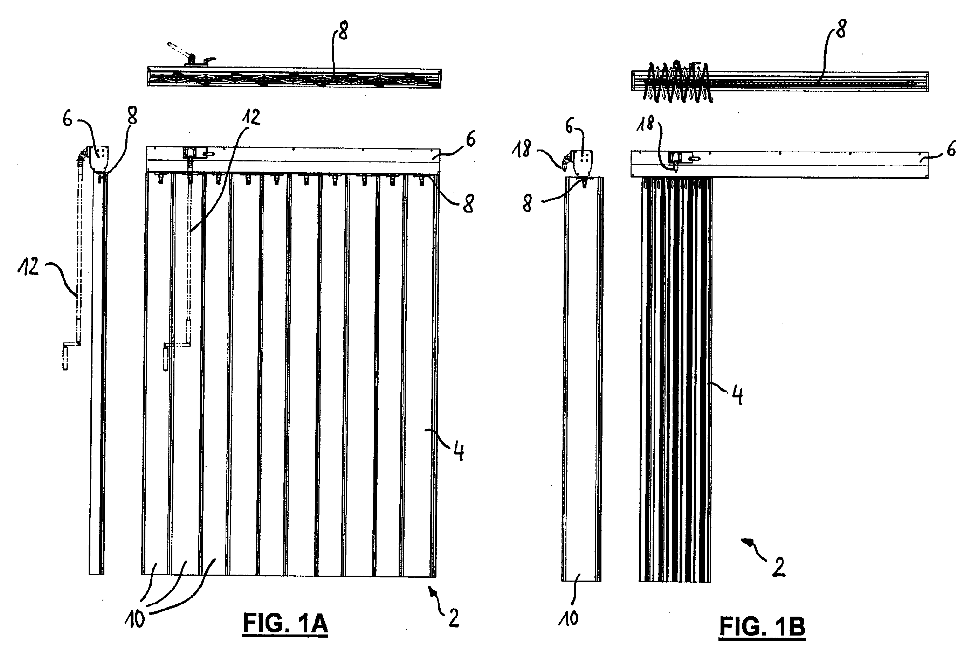

In den Figuren 1A und 1B ist eine Ausführungsform einer erfindungsgemäßen Strahlenschutz-Lamellenanordnung 2 gezeigt. In Figur 1A ist ein durch die erfindungsgemäße Strahlenschutz-Lamellenanordnung 2 gebildeter Faltvorhang 4 in geschlossener Position dargestellt, während in der Darstellung gemäß Figur 1B der Faltvorhang 4 geöffnet ist.

Die Strahlenschutz-Lamellenanordnung 2 weist eine in einem Gehäuse 6 angeordnete Führung 8 auf, an der mehrere beweglich angeordnete Strahlenschutz-Lamellen 10 angebracht sind. Die Strahlenschutz-Lamellenanordnung 2 ist von einem offenen Zustand gemäß Figur 1B durch Verschieben und Rotieren der Lamellen 10 in einen geschlossenen Zustand gemäß Figur 1A überführbar. Die unterschiedlichen Stellungen der einzelnen Lamellen sind insbesondere durch einen Vergleich der Draufsichten gemäß Figur 1A und 1B zu erkennen.

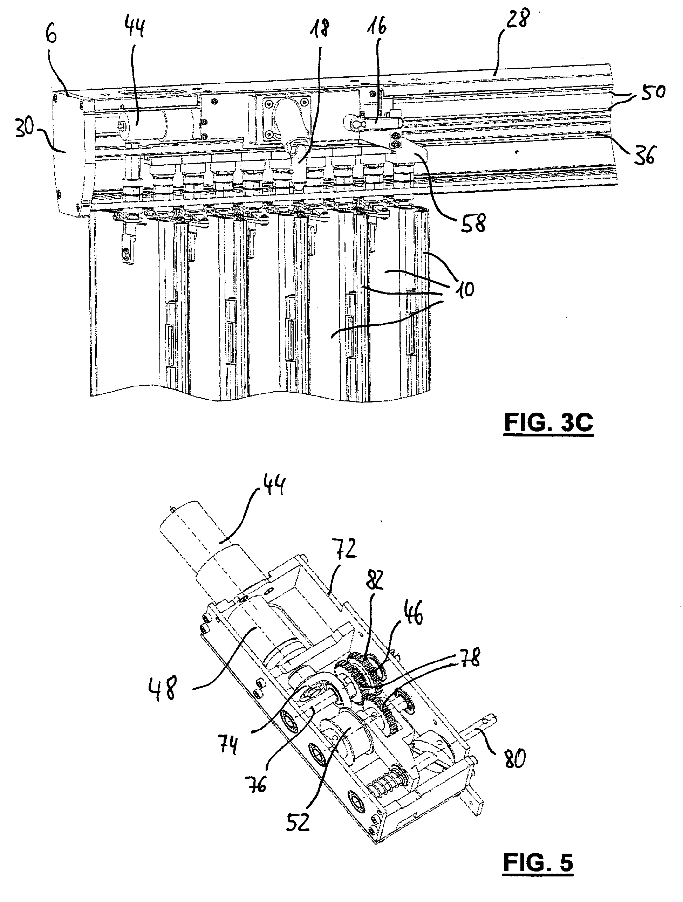

Gemäß einer bevorzugten Ausführungsform ist die Strahlenschutz-Lamellenanordnung 2 durch einen im Gehäuse 6 angeordneten Antriebsmechanismus, der insbesondere in Figur 5 näher gezeigt ist, betätigbar. Alternativ oder ergänzend dazu kann eine Handbetätigung, beispielsweise mit Hilfe einer Kurbel 12 realisiert sein. Vorzugsweise ist die Kurbel 12 lediglich als Notbetätigung vorgesehen, beispielsweise im Falle eines Stromausfalls.

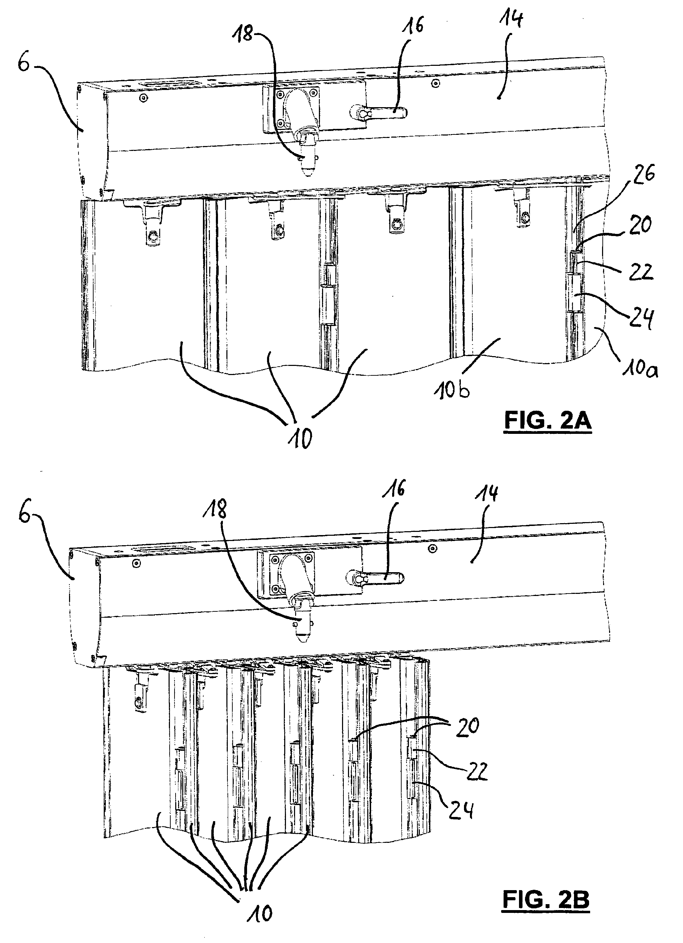

In den Figuren 2A und 2B ist die erfindungsgemäße Strahlenschutz-Lamellenanordnung 2, wie sie in den Figuren 1A und 1B gezeigt ist, in perspektivischer Form näher dargestellt. Dabei ist insbesondere zu erkennen, dass die Lamellen 10 sowohl in dem in Figur 2A gezeigten geschlossenen Zustand als auch in dem in Figur 2B gezeigten geöffneten Zustand eine im Wesentlichen geschlossene Strahlenschutzfläche bilden. Dies ist unabhängig von der Position und/oder der Orientierung der Lamellen 10 der Fall.

Ferner ist in den Figuren 2A und 2B an einem Seitenteil 14 des Gehäuses 6 ein Notentriegelungshebel 16 dargestellt, mit dem die Lamellenanordnung 2 von Automatik- in Handbetrieb umgestellt werden kann. In der dargestellten Position befindet sich die Anordnung beispielsweise im Automatikbetrieb. Wird der Hebel 16 beispielsweise um 90° aus der Horizontalen in die Vertikale gedreht oder alternativ z.B. um 180° geschwenkt, so kann die Anordnung auf Handbetrieb umgestellt werden. Ist Handbetrieb gewünscht, kann die Handkurbel 12 mit einem Antriebszapfen 18 in Eingriff gebracht werden, um den Faltvorhang 4 manuell zu betätigen. Alternativ dazu kann die Lamellenanordnung 4 per Hand verschoben werden.

Die einzelnen aneinander angrenzenden Lamellen 10 sind gemäß der Darstellung in den Figuren 2A und 2B gelenkig miteinander verbunden. Hierzu ist vorzugsweise ein lösbarer Gelenkbolzen 20 vorgesehen. Die einzelnen Gelenkbolzen 20 sind in Aufnahmeöffnungen 22 und 24 benachbarter Lamellen 10 eingesetzt, um diese gelenkig miteinander zu verbinden. In einer vorteilhaften Ausführungsform sind die Aufnahmeöffnungen 22 und 24 der Lamellen 10 im Lamellenprofil durchgehend vorgesehen, wobei lediglich im Bereich des Gelenkbolzens 20 eine Aussparung 26 vorgesehen ist, durch die der Gelenkbolzen 20 in die Aufnahmeöffnungen 22 und 24 eingeführt werden kann. Alternativ zu den Gelenkbolzen 20 kann auch ein z.B. dreiteiliges Kunststoffscharnier mit Zylinderstiften zum Einsatz kommen. Dann müssen die Lamellenprofile seitlich nicht bearbeitet werden.

Bei der in Figur 2A gezeigten Ausführungsform ist somit beispielsweise eine Lamelle 10a mit einer benachbarten Lamelle 10b wie folgt verbunden. Die Lamelle 10a weist ein im Wesentlichen durchgehendes Profil auf, in dem entlang der Längskanten die Aufnahmeöffnung 22 vorgesehen ist. Im Bereich der Gelenkverbindung ist das Profil zur Ausbildung einer Zugangsöffnung 26 ausgespart, zum Beispiel durch Abfräsen des Profils, so dass der Gelenkbolzen 20 in die Aufnahmeöffnung 22 der Lamelle 10a eingeführt werden kann. Die mit der Lamelle 10a zu verbindende Lamelle 10b weist ebenfalls ein entlang der Seitenkanten verlaufendes Aufnahmeprofil für den Gelenkbolzen 20 auf, das die Aufnahmeöffnung 24 definiert. Dieses Profil ist im Wesentlichen über einen Großteil der Länge der Lamelle 10b abgetragen und nur in den Gelenkbereichen vorhanden, in denen eine Aufnahmeöffnung 24 erforderlich ist. Mit anderen Worten bilden die beiden entlang der Seitenkanten der Lamellen 10a und 10b bereitgestellten Profile komplementär zueinander passende Vorsprünge, die ineinander greifen, so dass deren Aufnahmeöffnungen 22 und 24 axial zueinander ausgerichtet angeordnet werden können, um den Gelenkbolzen 20 aufzunehmen. Darüber hinaus ist vorzugsweise oberhalb der Aufnahmeöffnung 22 das Profil der Lamelle 10a über eine Länge entsprechend der Länge des Gelenkbolzens 20 abgetragen, so dass der Gelenkbolzen leicht zugänglich eingeführt werden kann.

Durch diesen Aufbau lassen sich auch bei montierter Anlage einzelne Lamellen 10 austauschen, indem die Gelenkbolzen 20 der betreffenden Lamellen entfernt werden und die jeweilige Lamelle von ihrer Aufnahme entfernt wird.

Wie in Figur 2A gezeigt, sind benachbarte Lamellen jeweils abwechselnd an einer Vorderseite und Hinterseite miteinander verbunden. Dementsprechend ist es unter Umständen vorteilhaft, die Gelenkbolzen auf der einen Seite des Faltvorhangs 4 beispielsweise im zumindest teilweise geschlossenen Zustand einzusetzen bzw. herauszunehmen, während die Gelenkbolzen 20 auf der gegenüberliegenden Seite beispielsweise vorzugsweise im vollständig geöffneten Zustand am besten zugänglich sind.

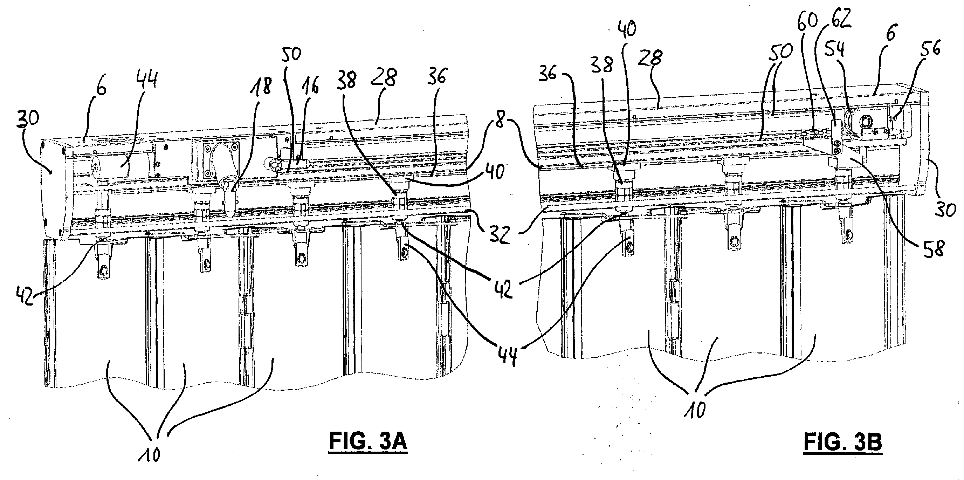

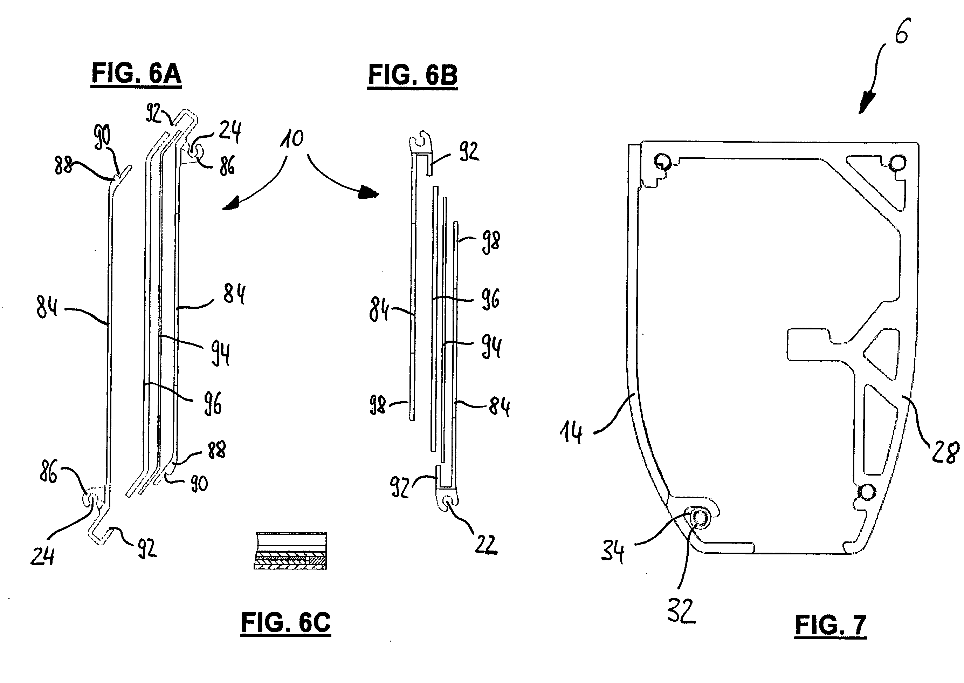

In den Figuren 3A und 3B ist insbesondere die Führung und der Antrieb des erfindungsgemäßen Faltvorhangs näher gezeigt. Figur 3A zeigt im Wesentlichen die Antriebsseite, während Figur 3B die der Antriebsseite gegenüberliegende Seite näher zeigt. Wie in Figur 3A ersichtlich, weist das Gehäuse ein Gehäuseprofil 28 entlang der Länge der Strahlenschutz-Lamellenanordnung auf, das zur Montage an einer Wand oder einer Decke ausgebildet ist. Das Gehäuseprofil 28 ist vorzugsweise ein Aluminiumstrangpressteil und kann in beliebiger Länge hergestellt werden. An den beiden Stirnenden des Gehäuseprofils 28 ist vorzugsweise jeweils eine Abdeckplatte 30 vorgesehen, die das Gehäuse 6 in Längsrichtung abschließen. Ferner weist das Gehäuse 6 eine vorzugsweise durchgehende Aufnahmestange 32 auf, die dazu vorgesehen ist, das Gehäuseseitenteil bzw. den Gehäusedeckel 14 aufzunehmen. Alternativ sind lediglich kurze Zapfen (nicht dargestellt) vorgesehen, um den Gehäusedeckel 14 aufzunehmen. Zapfen haben gegenüber einer durchgehenden Aufnahmestange 32 den Vorteil der einfacheren Zugänglichkeit bei Zusammenbau und Wartung. Der Gehäusedeckel 14 (siehe Figuren 2A, 2B und 7) wird vorzugsweise mit einer Nut 34 über die Aufnahmestange 32 geführt und dann nach oben geklappt, um den Gehäusedeckel 14 mit dem Gehäuseprofil 28 beispielsweise durch Verschraubung zu verschließen. Durch diese Anordnung, ist das Innere des Gehäuses 6 auf einfache Weise von der Seite zugänglich, wie dies in Figuren 3A und 3B gezeigt ist.

Im Gehäuse 6 ist zur Aufnahme der Lamellen 10 eine Führung 8 vorgesehen, die nachfolgend näher beschrieben wird. Die Führung 8 weist insbesondere eine im Wesentlichen entlang der Länge des Gehäuseprofils 28 verlaufende Führungsschiene 36 auf, an der die einzelnen Lamellen 10 mittels geeigneter Aufnahmen 38 sowohl in Längsrichtung verschiebbar als auch drehbar gelagert sind. Die Aufnahme 38 der beweglich geführten Lamellen 10 weist vorzugsweise ein auf der Führungsschiene 36 sitzendes Führungselement 40 auf, das vorzugsweise ein Linearlager enthält. Das Führungselement 40 ist wiederum vorzugsweise über ein Kugel- oder Kreuzgelenk 42 mit einer Lamellenaufnahme 44 verbunden. Um den auftretenden Kräften, insbesondere den hohen Scherkräften, Rechnung zu tragen, sind bevorzugt spezielle Lager für die Kreuz- oder Kugelgelenke vorgesehen. Durch diese Anbringung der einzelnen Lamellen 10 an der Führungsschiene 36 ist jede der Lamellen 10 entlang der Führungsschiene 36 in Längsrichtung beweglich, drehbar und durch das Kreuz- oder Kugelgelenk 42 auch schwenkbar gelagert.

Die in Figur 3A ganz links gezeigte Lamelle 10 ist entgegen den übrigen Lamellen 10 nicht entlang der Führungsschiene 36 beweglich gelagert, sondern direkt am Gehäuseprofil 28 stationär montiert. Diese Lamelle ist jedoch ebenfalls drehbar und durch das Kreuz- bzw. Kugelgelenk 42 schwenkbar angeordnet.

Zum Antrieb der erfindungsgemäßen Strahlenschutz-Lamellenanordnung 2 ist vorzugsweise ein Elektromotor 44 im Gehäuse 6 vorgesehen, der über ein in Figur 5 näher gezeigtes Getriebe 46 sowie eine zwischen dem Motor 44 und dem Getriebe 46 vorgesehene Rutschkupplung 48 ein Antriebselement 50, zum Beispiel eine Kette oder einen Zahnriemen, antreibt. Das Antriebselement 50 erstreckt sich von einer Abtriebswelle 52 des Getriebes im Wesentlichen entlang der Führungsschiene 36 bis zu einer Umlenkrolle 54, die in Figur 3B gezeigt ist. Im Bereich der Umlenkrolle 54 ist vorzugsweise eine Spanneinrichtung 56 vorgesehen, mit der das Antriebselement 50 auf eine geeignete Spannung eingestellt werden kann.

Zur Übertragung der Antriebsbewegung vom Motor 44 über das Antriebselement 50 auf die Lamellen 10 ist vorzugsweise die der feststehenden Lamelle (links in Figur 3A) gegenüberliegende entfernteste Lamelle 10 (rechts in Figur 3B) über einen Schlitten 58 mit dem Antriebselement 50 verbunden. Dies kann beispielsweise durch eine Klemmung 60 erfolgen. Der Schlitten 58 ist in der in Figur 3B gezeigten Ausführungsform mit dem Führungselement 40 verbunden, so dass er die der feststehenden Lamelle gegenüberliegende entfernteste Lamelle leichtgängig in Axialrichtung verschieben kann. Am Schlitten 58 ist vorzugsweise ein Vorsprung 62 vorgesehen, der beispielsweise mit einem Sensor zur Endabschaltung des Faltvorhangs 4 zusammenarbeitet, wenn der Faltvorhang 4 in seiner geschlossenen bzw. offenen Stellung ankommt.

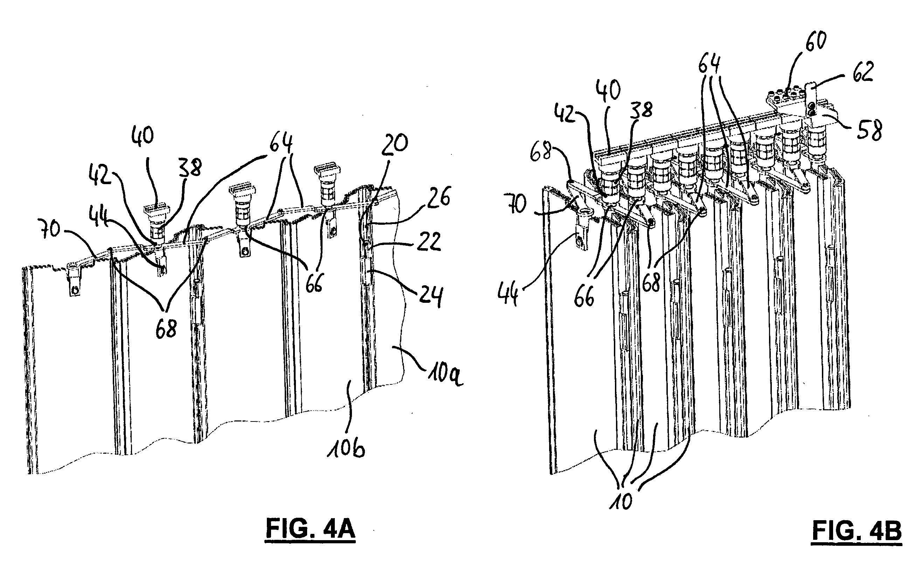

Die Übertragung der Antriebsbewegung von der durch den Schlitten 58 angetriebenen entfernten Lamelle 10 auf die übrigen beweglichen Lamellen wird nachfolgend unter Bezugnahme auf die Figuren 4A und 4B näher beschrieben. In den Figuren 4A und 4B sind die oberen Endabschnitte der Lamellen mit ihren Aufnahme- und Führungselementen näher gezeigt. Wie vorstehend bereits erläutert, ist am oberen Ende der Lamellen 10 ein Aufnahmeelement 44 angebracht, das über das Kreuz- oder Kugelgelenk 42 mit dem Führungselement 40 derart verbunden ist, dass jede der Lamellen eine Dreh- und Schwenkbewegung durchführen kann.

Das Führungselement 40 führt die einzelnen Lamellen 10 entlang der Führungsschiene 36. Um die Bewegung der Lamellen von der durch den Schlitten 58 in Bewegung versetzten Lamelle auf die übrigen beweglich gelagerten Lamellen 10 zu übertragen, sind diese in der dargestellten Ausführungsform durch Gelenkstreben 64 jeweils miteinander verbunden. Jede der Gelenkstreben 64 weist in ihrem Mittelbereich eine Durchgangsöffnung 66 auf, durch die jeweils eine Aufnahmeeinrichtung jeder Lamelle 10 verläuft, so dass die Gelenkstrebe 64 um die gleiche Drehachse rotierbar ist wie die jeweilige dazugehörige Lamelle. Ferner weisen die Gelenkstreben 64 der beweglich gelagerten Lamellen an ihren beiden gegenüberliegenden Enden Öffnungen 68 auf, an denen einander benachbart liegende Gelenkstreben 64 gelenkig miteinander verbunden sind.

Durch diese Anordnung sind sämtliche Lamellen 10 miteinander gekoppelt, so dass die durch den Schlitten 58 auf die am entferntest liegende Lamelle 10 übertragende Linearbewegung im Wesentlichen zeitgleich durch die Gelenkstreben 64 auf jede der Lamellen 10 übertragen wird, so dass alle Lamellen 10 im Wesentlichen zeitgleich durch den Antrieb in Längs- und Drehbewegung versetzt werden, um den Faltvorhang zu schließen.

Lediglich die nicht verschiebbare Lamelle 10, das heißt die dem Schlitten 58 gegenüberliegende Lamelle 10 im Bereich des Antriebs, erfährt dadurch eine Drehbewegung und keine Längsbewegung. Die Drehbewegung wird durch einen verkürzten Gelenkarm 70 eingebracht, der mit dem benachbart liegenden Gelenkarm 64 verbunden ist, wie dies in den Figuren 4A und 4B gezeigt ist.

Nachfolgend wird unter Bezugnahme auf die exemplarische Figur 5 ein bevorzugtes Getriebe 46 erläutert. Wie in Figur 5 gezeigt, ist an einem Getriebegehäuse 72 der Elektromotor 44 angebracht, der mit der im Gehäuse 72 liegenden Rutschkupplung 48 verbunden ist. Die Drehbewegung des Motors wird über die Rutschkupplung 48 mittels eines Kegelradgetriebes 74 auf eine Eingangswelle 76 übertragen. Die Eingangswelle 76 steht mit der Abtriebswelle 52 über ein Zahnradpaar 78 in Drehverbindung, wobei die Abtriebswelle 52 in eine geeignete Ausgangsdrehzahl versetzt wird. Im Falle der Betätigung des Notentriegelungshebels 16 wird das auf der Abtriebswelle 52 vorgesehene Zahnrad des Zahnradpaars 78 über eine Welle 80 durch die Drehung des Hebels 16 so verschoben, dass es mit einem Zahnrad 82 in Eingriff kommt. Das Zahnrad 82 ist auf einer Welle gelagert, die mit dem Zapfen 18 (Figur 3C) in Drehverbindung steht, so dass bei Drehung des Zapfens 18 beispielsweise durch die Handkurbel 12 die Abtriebswelle 52 in Drehbewegung versetzt werden kann, um den Faltvorhang über das Antriebselement 50 zu bewegen.

In den Figuren 6A, 6B und 6C sind Lamellen 10 zur Verwendung in der erfindungsgemäßen Strahlenschutz-Lamellenanordnung 2 näher gezeigt. Die in Figur 6A gezeigte Lamelle 10 stellt einen ersten in der erfindungsgemäßen Anordnung 2 verwendeten Lamellentyp dar. Dieser erste Lamellentyp weist im Wesentlichen zwei identische und punktsymmetrisch zueinander angeordnete Deckelemente 84 auf, die beispielsweise als Aluminiumstranggusselemente ausgebildet sein können. Die Deckelemente 84 weisen entlang ihrer Längskanten Abwinklungen in entgegengesetzte Richtungen auf. Im Bereich einer der Abwinklungen ist ein Profilvorsprung 86 vorgesehen, der die Aufnahmeöffnung 24 für den Gelenkbolzen 20 bildet.

In der gezeigten Ausführungsform ist im Bereich dieser Abwinklung das Deckelement 84 ferner U-förmig ausgebildet. An der gegenüberliegenden Abwinklung sind ein Anschlag 88 sowie eine Vertiefung 90 vorgesehen. Der Anschlag 88 und die Vertiefung 90 sind dazu ausgebildet, mit einem Endabschnitt 92 des U-förmigen Vorsprungs des anderen Deckelements 84 in Eingriff zu kommen. Dadurch können die beiden Deckelemente 84 miteinander verankert bzw. verriegelt werden und bilden einen Formschluss. Zwischen den beiden Deckelementen 84 ist im zusammengefügten Zustand ein Hohlraum gebildet, in den ein strahlungsundurchlässiges Material 94 und eine optionale Fülllage 96 eingebracht werden. Das strahlungsundurchlässige Material 94 kann beispielsweise im Fall von Röntgenstrahlen eine Bleilage sein. Das Füllmaterial 96 ist lediglich vorgesehen, um gegebenenfalls den Hohlraum aufzufüllen. Das heißt, wenn die strahlungsundurchlässige Lage 94 den gesamten Hohlraum ausfüllt, ist das Füllmaterial 96 obsolet. Die einzelnen Lagen 84, 94, 96, 84 werden vorzugsweise miteinander verklebt, um eine stabile Lamelle 10 bereitzustellen. Dies ist beispielsweise in Figur 6C im Querschnitt gezeigt.

In Figur 6B ist ein zweiter Lamellentyp 10 dargestellt, der wiederum aus zwei identischen Deckelementen 84 besteht, die zwischen einander einen Hohlraum definieren, in dem ein strahlenundurchlässiges Material 94 sowie ein optionales Füllmaterial 96 angeordnet sind. Die Deckelemente 84 gemäß Figur 6B sind ebenfalls mit U-förmigen Endabschnitten 92 ausgebildet, sie sind jedoch nicht wie in der in Figur 6A gezeigten Variante der ersten Lamellenart abgewinkelt. Am Ende der U-förmigen Endabschnitte ist wiederum jeweils ein Einhakvorsprung vorgesehen, der mit einer Nut 98 am Deckelement 84 in Eingriff kommt.

An den U-förmig ausgebildeten Endabschnitten 92 ist jeweils ein Profilvorsprung 86 entlang der Länge der Deckelemente ausgebildet, die die Aufnahmeöffnung 22 für den Gelenkbolzen 20 zur Verbindung benachbarter Lamellen 10 definieren. Die einzelnen Lagen 84, 94, 96, 84 des zweiten Lamellentyps 10 gemäß Figur 6B werden wiederum vorzugsweise miteinander verklebt, wobei die Vorsprünge in die Nuten 98 einhaken und zusätzlich vorzugsweise eine formschlüssige Verbindung bereitstellen.

Bei dem in den Figuren dargestellten Ausführungsbeispiel der erfindungsgemäßen Strahlenschutz-Lamellenanordnung wechseln benachbarte Lamellen 10 des ersten Typs gemäß Figur 6A und des zweiten Typs gemäß Figur 6B einander ab. Vorzugsweise ist der erste Lamellentyp etwas breiter ausgebildet als der zweite Lamellentyp, wie dies in Figuren 6A und 6B dargestellt ist. Die Breite der Lamellen liegt vorzugsweise im Bereich zwischen 20 und 900 mm, besonders bevorzugt zwischen 100 und 300 mm.

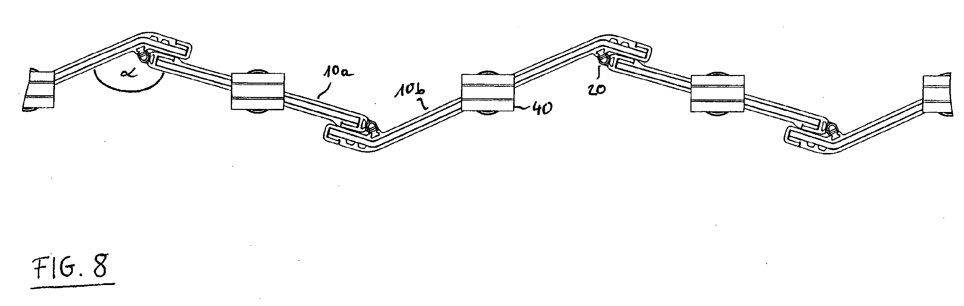

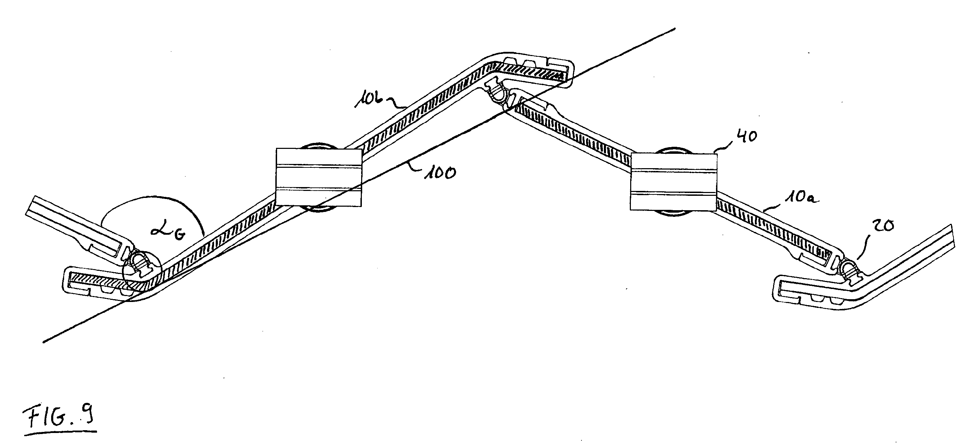

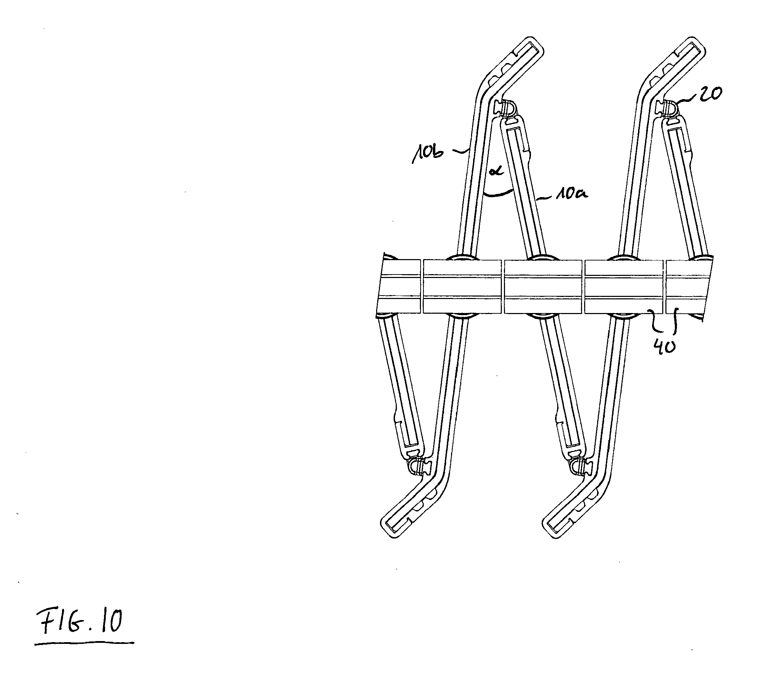

In den Figuren 8 bis 10 ist eine alternative Ausführungsform einer erfindungsgemäßen Strahlenschutz-Lamellenanordnung gezeigt. In Figur 8 ist ein durch die erfindungsgemäße Strahlenschutz-Lamellenanordnung gebildeter Faltvorhang in geschlossener Position dargestellt, während in der Darstellung gemäß Figuren 9 und 10 der Faltvorhang teilweise geschlossen bzw. geöffnet ist. Wie in Figur 10 deutlich zu erkennen ist, bilden jeweils zwei aneinander angrenzende Lamellen 10a und 10b im Zustand des geschlossenen Vorhangs einen spitzen Winkel α, der je nach Bauart und Anordnung des Gelenks 20 im völlig geschlossenen Zustand kleiner als 30°, bevorzugt kleiner als 15°, werden kann. Im vollständig geöffneten Zustand öffnet sich der Winkel α auf mehr als 120°, bevorzugt auf etwa 125° bis etwa 130°, in manchen Ausführungsformen jedoch auch bis zu 140°, 150° oder 160°.

Es ist ferner bevorzugt, mindestens eine Längsseite einer jeweiligen Lamelle abgewinkelt auszubilden. In der Ausführungsform der Figuren 8 bis 10 sind beide Längsseiten des einen Lamellentyps 10b abgewinkelt, wohingegen der zweite, an den ersten Lamellentyp angrenzende Lamellentyp 10a nicht abgewinkelt ist. Die Lamellen 10a und 10b wechseln sich dabei innerhalb des Vorhangs ab. Dabei korrespondieren vorzugsweise der Winkel des abgewinkelten Bereichs innerhalb der Lamelle und der zwischen angrenzenden Lamellen gebildete Maximalwinkel α so, dass bei vollständig geschlossenem Strahlenschutz (vgl. Figur 8) die angrenzende Lamelle im Wesentlichen parallel zu dem abgewinkelten Bereich zu liegen kommt.

Bevorzugt sind die Lamellen 10a und 10b derart überlappend angeordnet, dass ab einem Grenzwinkel α_{G} eine geschlossene Strahlenschutzfläche gebildet wird. In Figur 9 ist eine Situation dargestellt, in der genau dieser Grenzwinkel eingestellt ist: In dieser Situation gibt es genau eine Einstrahlrichtung für Strahlung 100, so dass diese zwischen dem strahlenundurchlässigen Material (schraffiert dargestellt) der Lamellen 10a und 10b hindurchtreten kann. Wird der Vorhang weiter geschlossen, d.h. wird α größer als der Grenzwinkel α_{G}, so kann keine Strahlung mehr durch den Vorhang gelangen. Bevorzugt wird bei Winkeln α größer als 100°, besonders bevorzugt bei Winkeln größer als 120°, eine in diesem Sinne geschlossene Strahlenschutzfläche gebildet. Wie aus Figur 9 ersichtlich wird, lässt sich der Winkelbereich, in dem vollständiger Schutz erzielt werden kann, dadurch anpassen, dass die Winkel innerhalb der Lamelle sowie die Länge der abgewinkelten Bereiche variiert wird. Auch die Dimensionen des Gelenks haben einen Einfluss.

Obwohl die erfindungsgemäße Strahlenschutz-Lamellenanordnung mit senkrecht vorgesehenen Lamellen 10 dargstellt ist, können die Lamellen 10 auch in waagrechter oder sonstiger Anordnung vorgesehen sein.

Die erfindungsgemäße Strahlenschutz-Lamellenanordnung ist aufgrund ihrer Gestaltung flexibel einsetzbar und kann insbesondere für Strahlenschutzanwendungen, beispielsweise als Raumteiler oder zur Abdeckung von Fensternischen, verwendet werden. Ein Strahlendurchtritt bzw. Strahlenaustritt zwischen den Lamellen 10 ist erfindungsgemäß effektiv verhindert, so dass ein flexibles, sicher arbeitendes System bereitgestellt wird.

## Patentansprüche

1. Strahlenschutz-Lamellenanordnung (2) zur Abschirmung von Röntgenstrahlung mit einer Führung (8) und mehreren entlang der Führung (8) beweglich angeordneten Strahlenschutz-Lamellen (10), wobei die Anordnung (2) von einem offenen Zustand durch Verschieben und Rotieren der Lamellen (10) in einen geschlossenen Zustand überführt werden kann,
**dadurch gekennzeichnet dass**
aneinander angrenzende Lamellen (10) einander überlappen und gelenkig miteinander verbunden sind und wobei die aneinander angrenzenden Lamellen einen variablen Winkel einschließen.

2. Strahlenschutz-Lamellenanordnung (2) nach Anspruch 1, wobei die aneinander angrenzenden Lamellen (10) einen Winkel einschließen, der zwischen 5° und 160°, bevorzugt zwischen 15° und 145°, variieren kann.

3. Strahlenschutz-Lamellenanordnung (2) nach Anspruch 2, wobei die Lamellen (10) derart überlappend angeordnet sind, dass bei Winkeln größer als 100°, bevorzugt bei Winkeln größer als 120°, eine geschlossene Strahlenschutzfläche gebildet wird.

4. Strahlenschutz-Lamellenanordnung (2) nach einem der Ansprüche 1 bis 3, wobei zumindest eine Längsseite der Lamellen (10) abgewinkelt ist.

5. Strahlenschutz-Lamellenanordnung (2) nach einem der Ansprüche 1 bis 4, wobei die Anordnung Lamellen (10) eines ersten Typs und Lamellen (10) eines zweiten Typs aufweist und wobei die Lamellen des ersten Typs an beiden Längsseiten abgewinkelt sind und die Lamellen des zweiten Typs im Wesentlichen plan sind.

6. Strahlenschutz-Lamellenanordnung (2) nach einem der Ansprüche 1 bis 5, wobei aneinander angrenzende Lamellen (10) durch lösbare Gelenkbolzen (20) miteinander verbunden sind.

7. Strahlenschutz-Lamellenanordnung (2) nach einem der Ansprüche 1 bis 6, wobei jede der Lamellen (10) zwei Deckelemente (84) aufweist, zwischen denen ein Hohlraum zur Aufnahme eines strahlenundurchlässigen Materials (94) und eines optionalen Füllmaterials (96) gebildet ist.

8. Strahlenschutz-Lamellenanordnung (2) nach Anspruch 7, wobei die zwei Deckelemente (84) im Wesentlichen identisch sind und punktsymmetrisch zueinander angeordnet sind.

9. Strahlenschutz-Lamellenanordnung (2) nach Anspruch 6 oder 7, wobei die Deckelemente (84), das strahlenundurchlässige Material (94) und das optionale Füllmaterial (96) miteinander verklebt sind und wobei die Deckelement (84) vorzugsweise zusätzlich formschlüssig miteinander verbunden sind.

10. Strahlenschutz-Lamellenanordnung (2) nach einem der Ansprüche 1 bis 9, wobei sich aneinander angrenzende Lamellen (10) in Form und/oder Größe unterscheiden.

11. Strahlenschutz-Lamellenanordnung (2) nach einem der Ansprüche 1 bis 10, wobei die Führung (8) als Aufhängung für die Lamellen (10) ausgebildet ist.

12. Strahlenschutz-Lamellenanordnung (2) nach einem der Ansprüche 1 bis 11, wobei die Lamellen (10) jeweils über ein Kugel- oder Kreuzgelenk (42) an der Führung (8) angebracht sind.

13. Strahlenschutz-Lamellenanordnung (2) nach einem der Ansprüche 1 bis 12, wobei das Verschieben und Rotieren der Lamellen (10) mit einem Motor (44) und alternativ manuell erfolgt.

14. Röntgengerät in Kombination mit einer Strahlenschutz-Lamellenanordnung (2) nach einem der Ansprüche 1 bis 13 mit einer Einrichtung zur Kommunikation zwischen Röntgengerät und Strahlenschutz-Lamellenanordnung (2), wobei das Röntgengerät nur dann betriebsbereit ist, wenn die Anordnung im geschlossenen Zustand ist.

## Claims

1. A radiation protective slat arrangement (2) for shielding X-ray radiation comprising a guide (8) and several radiation protective slats (10) movably arranged along the guide (8), wherein the arrangement (2) can be converted from an open state into a closed state by displacing and rotating the slats (10),
**characterised in that**
adjacent slats (10) overlap each other and are articulated to each other and wherein the adjacent slats enclose a variable angle.

2. The radiation protective slat arrangement (2) according to claim 1, wherein the adjacent slats (10) enclose an angle which can vary between 5° and 160°, preferably between 15° and 145°.

3. The radiation protective slat arrangement (2) according to claim 2, wherein the slats (10) are arranged to overlap each other such that a closed radiation protective area is formed when the angle is greater than 100°, preferably greater than 120°.

4. The radiation protective slat arrangement (2) according to any one of claims 1 to 3, wherein at least one longitudinal side of the slats (10) is angled.

5. The radiation protective slat arrangement (2) according to any one of claims 1 to 4, wherein the arrangement comprises slats (10) of a first type and slats (10) of a second type and wherein the slats of the first type are angled on both longitudinal sides and the slats of the second type are substantially planar.

6. The radiation protective slat arrangement (2) according to any one of claims 1 to 5, wherein adjacent slats (10) are connected to each other by detachable pivot pins (20).

7. The radiation protective slat arrangement (2) according to any one of claims 1 to 6, wherein each of the slats (10) comprises two cover elements (84) between which a cavity is formed for accommodating a radiopaque material (94) and an optional filling material (96).

8. The radiation protective slat arrangement (2) according to claim 7, wherein the two cover elements (84) are essentially identical and are arranged point-symmetrically with respect to each other.

9. The radiation protective slat arrangement (2) according to claim 6 or 7, wherein the cover elements (84), the radiopaque material (94) and the optional filling material (96) are glued to one another and wherein the cover elements (84) preferably are additionally connected to each other with positive locking.

10. The radiation protective slat arrangement (2) according to any one of claims 1 to 9, wherein adjacent slats (10) differ in shape and/or size.

11. The radiation protective slat arrangement (2) according to any one of claims 1 to 10, wherein the guide (8) is configured as suspension for the slats (10).

12. The radiation protective slat arrangement (2) according to any one of claims 1 to 11, wherein each of the slats (10) is attached to the guide (8) by a ball-and-socket or a universal joint (42).

13. The radiation protective slat arrangement (2) according to any one of claims 1 to 12, wherein the displacement and rotation of the slats (10) is performed with a motor (44) and alternatively manually.

14. An X-ray apparatus in combination with a radiation protective slat arrangement (2) according to any one of claims 1 to 13 comprising a means for the communication between the X-ray apparatus and the radiation protective slat arrangement (2), wherein the X-ray apparatus is only operable when the arrangement according to the invention is in its closed state.

## Revendications

1. Dispositif d'écran radioprotecteur à lamelles (2), imperméable aux rayons X, comportant une glissière (8) et plusieurs lamelles radioprotectrices (10) mobiles le long de la glissière (8), où ledit dispositif (2) peut être commuté d'un état d'ouverture à un état de fermeture par translation et rotation des lamelles (10), **caractérisé en ce que** les lamelles (10) adjacentes se chevauchent l'une l'autre et sont reliées de manière articulée l'une à l'autre, et **en ce que** les lamelles adjacentes forment un angle variable entre elles.

2. Dispositif d'écran radioprotecteur à lamelles (2) selon la revendication 1, où les lamelles (10) adjacentes forment entre elles un angle pouvant varier entre 5° et 160°, préférentiellement entre 15° et 145°.

3. Dispositif d'écran radioprotecteur à lamelles (2) selon la revendication 2, où les lamelles (10) se chevauchent de manière à former une surface radioprotectrice en cas d'angle supérieur à 100°, préférentiellement en cas d'angle supérieur à 120°.

4. Dispositif d'écran radioprotecteur à lamelles (2) selon l'une des revendications 1 à 3, où au moins une longueur des lamelles (10) est pliée.

5. Dispositif d'écran radioprotecteur à lamelles (2) selon l'une des revendications 1 à 4, où le dispositif comprend des lamelles (10) d'un premier type et des lamelles (10) d'un deuxième type, et où les lamelles du premier type sont pliées sur les deux longueurs et les lamelles du deuxième type sont sensiblement planes.

6. Dispositif d'écran radioprotecteur à lamelles (2) selon l'une des revendications 1 à 5, où les lamelles (10) adjacentes sont assemblées par des goupilles d'articulation (20) amovibles.

7. Dispositif d'écran radioprotecteur à lamelles (2) selon l'une des revendications 1 à 6, où chaque lamelle (10) comporte deux éléments de couverture (84) entre lesquels une cavité est formée pour la réception d'un matériau impénétrable aux radiations (94) et d'un matériau de remplissage (96) facultatif.

8. Dispositif d'écran radioprotecteur à lamelles (2) selon la revendication 7, où les deux éléments de couverture (84) sont sensiblement identiques et ponctuellement symétriques entre eux.

9. Dispositif d'écran radioprotecteur à lamelles (2) selon la revendication 6 ou la revendication 7, où les éléments de couverture (84), le matériau impénétrable aux radiations (94) et le matériau de remplissage (96) facultatif sont collés entre eux, et où les éléments de couverture (84) sont préférentiellement assemblés entre eux par correspondance de forme.

10. Dispositif d'écran radioprotecteur à lamelles (2) selon l'une des revendications 1 à 9, où des lamelles (10) adjacentes se distinguent par leur forme et/ou par leurs dimensions.

11. Dispositif d'écran radioprotecteur à lamelles (2) selon l'une des revendications 1 à 10, où la glissière (8) est réalisée comme suspension pour les lamelles (10).

12. Dispositif d'écran radioprotecteur à lamelles (2) selon l'une des revendications 1 à 11, où chaque lamelle (10) est montée sur la glissière (8) au moyen d'une articulation à rotule ou d'une articulation à cardan (42).

13. Dispositif d'écran radioprotecteur à lamelles (2) selon l'une des revendications 1 à 12, où la translation et la rotation des lamelles (10) sont réalisées par un moteur (44), et manuellement de manière alternative.

14. Appareil radiographique combiné à un dispositif d'écran radioprotecteur à lamelles (2) selon l'une des revendications 1 à 13, comportant un dispositif de communication entre ledit appareil radiographique et le dispositif d'écran radioprotecteur à lamelles (2), ledit appareil radiographique n'étant prêt à fonctionner que lorsque le dispositif est en état de fermeture.
